(19) **Europäisches Patentamt** / **European Patent Office** / **Office européen des brevets**

(11) **EP 4 069 117 B1**

(12) **EUROPEAN PATENT SPECIFICATION**

(45) Date of publication and mention
of the grant of the patent:
**15.05.2024 Bulletin 2024/20**

(21) Application number: **20819658.4**

(22) Date of filing: **30.11.2020**

(51) International Patent Classification (IPC):
**A61B 18/12** *(2006.01)* **H03K 3/335** *(2006.01)*

(52) Cooperative Patent Classification (CPC):
**A61B 18/1206; H03K 3/53;** A61B 2018/126;
A61B 2018/1286

(86) International application number:
**PCT/EP2020/083975**

(87) International publication number:
**WO 2021/110605 (10.06.2021 Gazette 2021/23)**

(54) **PULSE GENERATING CIRCUIT, AND ELECTROSURGICAL GENERATOR INCORPORATING THE SAME**

IMPULSERZEUGUNGSSCHALTUNG UND ELEKTROCHIRURGISCHER GENERATOR MIT SELBIGEM

CIRCUIT DE GÉNÉRATION D'IMPULSIONS ET GÉNÉRATEUR ÉLECTROCHIRURGICAL L'INCORPORANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.12.2019 GB 201917695**

(43) Date of publication of application:
**12.10.2022 Bulletin 2022/41**

(73) Proprietor: **Creo Medical Limited**
**Chepstow, Monmouthshire NP16 5UH (GB)**

(72) Inventors:
• **HANCOCK, Christopher Paul**
**Chepstow NP16 5UH (GB)**
• **DAVIES, Ilan**
**Chepstow NP16 5UH (GB)**
• **HODGKINS, George**
**Chepstow NP16 5UH (GB)**

(74) Representative: **Mewburn Ellis LLP**
**Aurora Building**
**Counterslip**
**Bristol BS1 6BX (GB)**

(56) References cited:
**US-A- 3 504 199 US-A- 5 675 275**
**US-A1- 2019 216 533**

• **Tampieri Daniele: "Avalanche transistor", , 30 November 2019 (2019-11-30), XP055772227, Wikipedia Retrieved from the Internet: URL:https://en.wikipedia.org/w/index.php?t itle=Avalanche_transistor&oldid=928674358 [retrieved on 2021-02-04]**
• **WERNER B HERDEN: "Application of avalanche transistors to circuits with a long mean time to failure", IEEE TRANSACTIONS ON INSTRUMENTATION AND MEASUREMENT, IEEE, USA, vol. IM-25, no. 2, 1 June 1976 (1976-06-01), pages 152-160, XP011464300, ISSN: 0018-9456, DOI: 10.1109/TIM.1976.6312331**

**Description**

TECHNICAL FIELD

The present invention relates to a pulse generating circuit for an electrosurgical generator and to an electrosurgical generator for generating a waveform suitable for causing electroporation of biological tissue.

BACKGROUND TO THE INVENTION

Electrosurgical generators are pervasive throughout hospital operating theatres, for use in open and laparoscopic procedures, and are also increasingly present in endoscopy suites. In endoscopic procedures the electrosurgical accessory is typically inserted through a lumen inside an endoscope. Considered against the equivalent access channel for laparoscopic surgery, such a lumen is comparatively narrow in bore and greater in length.

WO 2019/185331 A1 discloses an electrosurgical generator capable of supplying energy in a waveform that causes electroporation in biological tissue. The electrosurgical generator may comprise an electroporation waveform supply unit that is integrated with means for generating microwave electromagnetic signals and radiofrequency electromagnetic signals for treatment. The electrosurgical generator may be configured to deliver different types of energy along a common feed cable. The electroporation waveform supply unit comprises a DC power supply and a DC pulse generator. The DC power supply may include a DC-DC converter for up-converting a voltage output by an adjustable voltage supply. Each DC pulse may have a duration in the range 1 ns to 10 ms and a maximum amplitude in the range 10 V to 10 kV.

Tampieri Daniele: "Avalanche transistor", published on Wikipedia on November 30th 2019, describes a pulse generating circuit for an electrosurgical generator comprising a voltage source connected to a load and an avalanche transistor as switching element, an open circuit coaxial transmission line connected between the switching element and the voltage source to be charged by the voltage source when the switching element is in an OFF state and to be discharged when the switching element is in an ON state and a trigger pulse generator to generate a trigger pulse to activate the avalanche transistor.Werner B Herden: "Application of avalanche transistors to circuits with a long mean time to failure",IEEE Transactions on instrumentation and measurement, vol. IM-25, no. 2, 1 June 1976, pages 152-160, describes a pulse generating circuit with the switching element comprising a plurality of series connected avalanche transistors.

In recent years there have been numerous developments of ultrashort electric field pulse generators [1]. Ultrashort electric field pulses in the nanosecond regime have numerous applications. Applications includes: measurement of particles, photography, ultra-wideband radar detection and medical application to name a few [2]-[3].

There are numerous methods of generating high amplitude, nanosecond pulsed electric field with a rise and fall time of 2 ns. Traditionally, coaxial transmission line-based implementations, such as Blumlein, in correlation with spark-gap, Marx bank, or diode and laser opening switch techniques have been used to generate high-voltage nanosecond pulses [1].

SUMMARY OF THE INVENTION

The invention is defined by the appended independent claim. Preferred embodiments of the invention are illustrated in the dependent claims. At its most general the present invention provides a pulse generating circuit for an electrosurgical generator, which is configured to generate high voltage pulses having a duration less than 10 ns suitable for causing electroporation of biological cells. In particular, the pulse generating circuit disclosed herein may be suitable for generating bipolar pulses that exhibit a 'flat-top' profile, i.e. having steep (e.g. less than 2 ns) rise and fall times, with minimal ringing. As explained in more detail below, this can be achieved through an open circuit transmission line technique in conjunction with placing a pair of load outputs (for positive and negative pulses) in suitable locations relative to the transmission line and a fast switching element.

According to the invention there is provided a bipolar pulse generating circuit for an electrosurgical generator, the bipolar pulse generating circuit comprising: a voltage source connectable to a load via a switching element; a coaxial transmission line having an inner conductor separated from an outer conductor by a dielectric material, wherein the inner conductor has a first end connected between an input of the switching element and the voltage source and a second end in an open circuit condition, whereby the coaxial transmission line is charged by the voltage source when the switching element is in an OFF state and to be discharged when the switching element is in an ON state; a first output connectable to the load, wherein the first output is located between an output of the switching element and ground to support a positive pulse when the coaxial transmission line discharges; and a second output connectable to the load, wherein the second output is located between the outer conductor of the coaxial transmission line and ground to support a negative pulse when the coaxial transmission line discharges, wherein the impedance of the coaxial transmission line is configured to match a sum of (i) the impedance the switching element, (ii) the impedance of the load at the first output, and (iii) the impedance of the load at the second output, and a delay line connected either in the first output or the second output, whereby supply of the positive pulse and negative pulse at the first output and the second output occurs sequentially. With this structure, the circuit can generate a bipolar pulse of ultrashort (e.g. less than 10 ns) duration in which the positive and negative parts of the pulse are symmetrical. Matching the impedances ensures that reflection is minimised or eliminated. This circuit configuration can yield a flat top pulse (due to the matched impedance condition) having a short duration (controlled by the length of the coaxial transmission line).

For example, a delay line may be connected between the outer conductor of the coaxial transmission line and the second output. The length of the delay line is selectable (or adjustable) to control the amount by which the negative pulse appears at the second output relative to the beginning of the positive pulse. It is possible to include a delay line connected at each of the first output and second output in order to provide full control of the profile of the output bipolar waveform. In particular, it may be desirable to provide a delay line having an adjustable length, so that the separation of the positive and negative pulses can be independently controlled. The delay line may have any suitable structure. For example, it may be another length of coaxial transmission line.

The switching element may comprise: a plurality of series connected avalanche transistors; and a trigger pulse generator configured to generate a trigger pulse to activate the plurality of series connected avalanche transistors. This enables the positive and negative pulses to have ultrashort rise and fall times and an amplitude suitable for electroporation due to the cascading effect of the series connected avalanche transistors. In particular, the amplitude of the output may be 500 V or more, e.g. 1 kV or more, without exceeding the collector-base breakdown voltage across any of the plurality of series connected avalanche transistors.

The coaxial transmission line may have a length selected to provide a line delay equal to or less than 5 ns. The pulse duration is twice the line delay, so the output pulse may have duration equal to or less than 10 ns.

The coaxial transmission line may be charged by the voltage source through a resistor having a high impedance, e.g. 1 MΩ. The circuit may thus be considered as comprises a first loop when the switching element is in an OFF state and a second loop when the switching element is in an ON state. In the first loop, current flows from the voltage source through the resistor to charge the coaxial transmission line. In the second loop, current flows from the coaxial transmission line through the switching element to the load.

The trigger pulse may comprise a TTL signal. The trigger pulse generator may be any source suitable for generating such a signal, e.g. a microprocessor or the like. The trigger pulse may have a voltage less than the emitter-base breakdown voltage of each of the plurality of avalanche transistors. The duration of the trigger pulse may be longer than the duration of the pulse from the coaxial transmission line, to ensure that the switching element is in an ON state for long enough for the coaxial transmission line to completely discharge. In one example, the trigger pulse has a voltage of 5 V and a duration of 600 ns.

The trigger pulse generator may be connected to the plurality of series connected avalanche transistors via a transformer. This means that the trigger signal is floating between the base and emitter, and is therefore independent of the voltage through the transistor and on to the load. In one example, the trigger pulse may be applied between the collector and emitter of a first transistor of the plurality of series connected avalanche transistors. The first transistor may be the transistor that is furthest from the coaxial transmission line.

A diode may be connected in parallel with each of the plurality of series connected avalanche transistors to clamp the voltage across each transistor to less than its collector-base breakdown voltage. This protects the transistors.

Each transistor in the plurality of series connected avalanche transistors may be identical so that a voltage of the voltage source is divided evenly between the transistors.

As mentioned above, the present invention is particularly suited for use in electrosurgery. The load may therefore comprise an electrosurgical instrument capable of delivered a monopolar pulse for electroporation of biological tissue.

The invention provides an electrosurgical generator having a pulse generating circuit as set out above. The pulse generating circuit may be configured to generate an electroporation waveform, i.e. a burst of energy suitable for causing electroporation of biological tissue. The electroporation waveform may comprise one or more rapid high voltage pulses. Each pulse may have a pulse width in a range from 1 ns to 10 μs, preferably in the range from 1 ns to 10ns, although the invention need not be limited to this range. Shorter duration pulses (e.g. equal to or less than 10 ns) may be preferred for reversible electroporation.

Preferably the rise time of each pulse is equal to or less than 90% of the pulse duration, more preferably equal to or less than 50% of the pulse duration, and most preferably equal to or less than 10% of the pulse duration.

Each pulse may have an amplitude in the range 10 V to 10 kV, preferably in the range 1 kV to 10 kV. Each pulse may be positive pulse from a ground potential.

The electroporation waveform may be a single pulse or a plurality of pulses, e.g. a period train of pulses. The waveform may have a duty cycle equal to or less than 50%, e.g. in the range 0.5% to 50%.

BRIEF DESCRIPTION OF THE DRAWINGS

Embodiments of the invention are discussed below with reference to the accompanying drawings, in which:

Fig. 1 is a schematic diameter that illustrates the principle of a discharge line generator with an ideal switch;
Fig. 2A is a graph showing a voltage waveform at (i) the transmission line, and (ii) the load in Fig. 1;
Fig. 3A is a schematic diagram representing the open circuit transmission line of Fig. 1 in a DC model;
Fig. 3B is a schematic diagram representing the open circuit transmission line of Fig. 1 in a transmission line model;
Fig. 4 is a schematic diagram of showing the open circuit transmission line of Fig. 1 with an avalanche transistor to generate a positive ultrashort electric field pulse;
Fig. 5 is a diagram of a simulated LTSpice circuit of a monopolar ultrashort electric field pulse generator;
Fig. 6 is a graph showing pulses of various durations generated from the LTSpice circuit of Fig. 5;
Fig. 7 is a monopolar positive pulse observed with a matched 35 Q load, from circuit in Fig. 5;
Fig. 8 is a schematic diagram of showing the open circuit transmission line of Fig. 1 with an avalanche transistor to generate a negative ultrashort electric field pulse;
Fig. 9 is a diagram of a simulated LTSpice circuit of a monopolar ultrashort electric field pulse generator configured to generate a negative pulse;
Fig. 10 is a monopolar negative pulse observed with a matched 35 Q load, from circuit in Fig. 9;
Fig. 11A is a diagram of a simulated LTSpice circuit of a bipolar ultrashort electric field pulse generator without delay lines;
Fig. 11B is a diagram of a simulated LTSpice circuit of a bipolar ultrashort electric field pulse generator with delay lines before the load;
Fig. 12A is a graph depicting voltage observed at various points in the circuit of Fig. 11A; and
Fig. 12B is a graph depicting voltage observed at various points in the circuit of Fig. 11A.

DETAILED DESCRIPTION, FURTHER OPTIONS AND PREFERENCES

Generation of ultra-short pulses is possible by using an open circuit coaxial transmission line as a high-Q storage element consisting of distributed series of inductors and shunt capacitors with minimal resistance and shunt conductance. Discharging an open ended delay line via a fast switching element provides a means of producing a 'flat-top' rectangular pulse with steep fall times of less than 2 ns in a simple and affordable manner. The co-axial transmission line with a characteristic impedance, $Z_0$, a length of $l$, and a dielectric constant $\varepsilon_r$, is charged to a voltage level, $V_{cc}$, through a high impedance resistor, $R_c$. The line will have and associated delay time $T$ given by the following equation:

$$T = \frac{l\sqrt{\varepsilon_r}}{c}$$

where c is the speed of light ($2.99 \times 10^8$ m/s).
It follows from this that the pulse duration associated with the transmission line is:

$$2T = 2\frac{l\sqrt{\varepsilon_r}}{c}$$

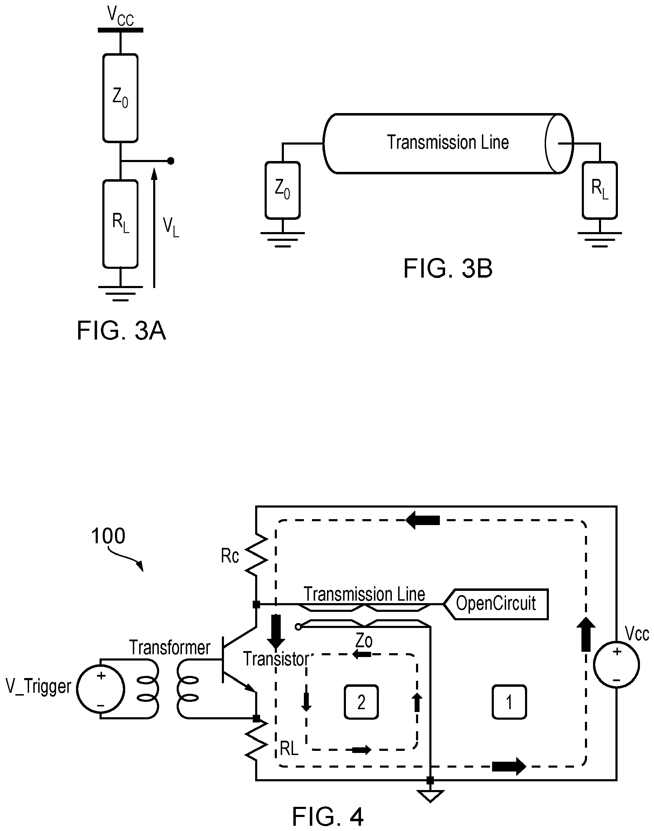

An ultrashort electric field pulse can be generated on a load, $R_L$, by discharging the transmission line through $R_L$ by closing a switching element. The switching element determines the rise time of the ultrashort electric field pulse whilst the transmission line determines the pulse duration (or width) and the fall time.
As explained above, the duration of the pulse at the load will be twice the associated delay time of the transmission line.

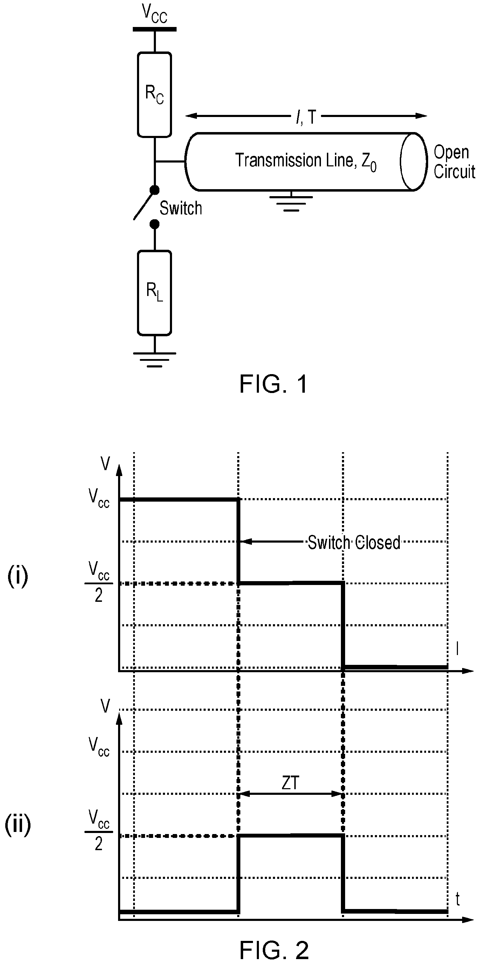

Fig. 1 illustrates the principle of an open circuit transmission line technique with an ideal switch as the switching element.
Fig. 2 shows the voltage waveforms obtained from the system of Fig. 1 at (i) the transmission line $Z_0$ and (ii) load $R_L$.
The relationship between the characteristic impedance of the transmission line $Z_0$ and the load $R_L$ is integral to the performance of an open circuit coaxial transmission line technique in two ways, which can be understood by modelling the configuration using direct circuit (DC) theory and transmission line theory.
In DC theory, the relationship between $Z_0$ and $R_L$ imitates a potential divider, as shown in Fig. 3A. Their relationship determines the pulse amplitude at the load $V_L$:

$$V_L = \left(\frac{R_L}{R_L + Z_0}\right) V_{cc}$$

If the impedance $Z_0$ is the same as $R_L$, the maximum amplitude of the pulse at the load, $V_{Lmax}$, will be half the voltage the to which the transmission line is charged:

$$if \ R_L = Z_0, \quad V_{Lmax} = \frac{V_{cc}}{2}$$

Using a transmission line model, the system can be represented as shown in Fig. 3B. In this model, the relationship between $Z_0$ and $R_L$ determines the reflection coefficient, and therefore the pulse shape at the load. If $R_L$ is the same as $Z_0$, the reflection coefficient will be zero and no secondary pulse or reflection of the primary pulse will be seen at the load:

$$\Gamma = \left(\frac{R_L - Z_0}{R_L + Z_0}\right) \qquad \therefore if \ R_L = Z_0, \ \ \Gamma = 0$$

Thus, the relationship of $Z_0$ and $R_L$ determine two key aspects of the pulse at a load: (i) the pulse amplitude, and (ii) pulse shape (caused by any reflection). It follows from the analysis above, that the best pulse shape and parameters, the characteristic impedance of the transmission line $Z_0$ and the load $R_L$ should match.

Other features of the pulse are controlled by other parameters of the circuit. For example, the pulse risetime is determined by the behavioural of the switching element, whilst the pulse width is determined by the length of the transmission line, as discussed above.

This switching element is preferably provided by a stacked array of avalanche transistors. An avalanche transistor is known to provide reliable and repeatable high-speed switching of high voltages with rise times as low as 300 ps, which can be achieved in practice if microwave component layout techniques are considered when the circuit are implemented. Avalanche transistors utilize the negative-resistance characteristics region of bipolar junction transistors, which result from operation in the common-emitter breakdown region. The avalanche region lies between collector emitter ($V_{CEO}$) and collector base ($V_{CBO}$) voltage when the base current $I_B$ = 0 A and emitter current $I_E$ = 0 A.

Fig. 4 is a schematic diagram of a pulse generating circuit 100 that utilises an open circuit transmission line technique in combination with an avalanche transistor as a fast switching element. The circuit function is based on the discharge of the open-circuit transmission line across an avalanche transistor into a load $R_L$.

A single avalanche transistor circuit can be configured to have a bi-stable operation, where the maximum pulse amplitude at the output is limited to half the value of the transistor's collector-emitter breakdown voltage, $BV_{CES}$, if $Z_0$ = $R_L$. A supply voltage $V_{cc}$ above the transistor's $BV_{CES}$ would permanently breakdown and damage the avalanche transistors as a switching element.

Initially, energy is stored in a co-axial transmission line via a small current flow in loop 1. A positive trigger on the base of the transistor will suddenly switch the transistor 'on'. The energy stored in the transmission line will simultaneously be released as a high current along loop 2, producing a pulse on $R_L$. The width of the trigger on the base is longer than $2T$, i.e. the required pulse width at the load.

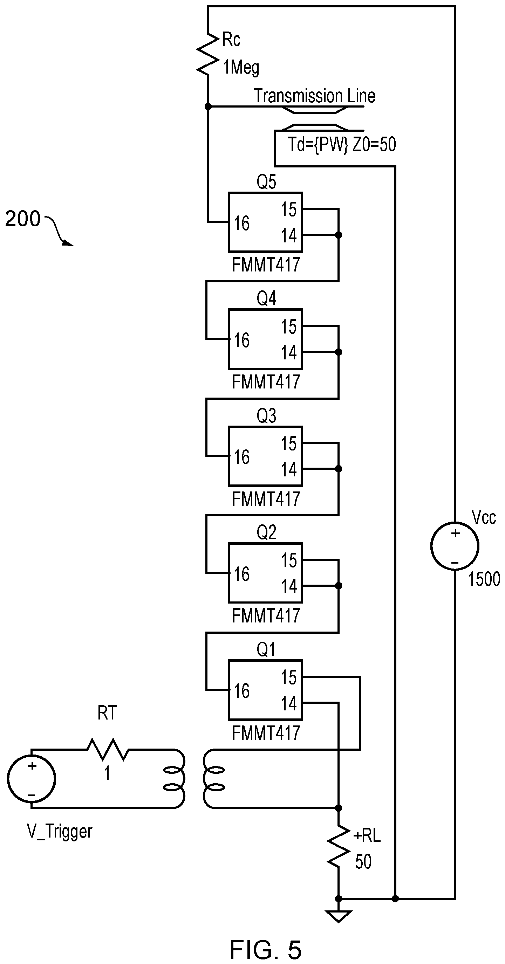

Fig. 5 shows a pulse generation circuit 200 that is an embodiment of the invention. The pulse generation circuit 200 is similar to the circuit shown in Fig. 4, except that in place of the single avalanche transistor, there is a plurality (five in this example) of series-connected avalanche transistors. The plurality of series-connected avalanche transistors effectively operate in combination as a single avalanche transistor. This means that the discharge of the open-circuit transmission line is across the stacked transistors to the load, thereby resulting in a cascade effect that causes a proportionally higher pulse amplitude at the load. In this example, each of the avalanche transistors is identical so that the supply voltage $V_{cc}$ is equally distributed across each of the avalanche transistor in the series chain.

In this arrangement, the maximum pulse amplitude that can be generated is dependent on the number of stacked avalanche transistor $n$. The number of avalanche transistors required to generate a specific pulse amplitude $V_L$ can be expressed as

$$V_L = nBV_{CBO}\left(\frac{R_L}{R_L + Z_0}\right)$$

where $BV_{CBO}$ is the collector-base breakdown voltage of each avalanche transistor. If $R_L = Z_0$, a maximum pulse amplitude $V_{Lmax}$ can thus be expressed as

$$V_{Lmax} = \frac{nBV_{CBO}}{2}$$

In the pulse generating circuit 200 five FMMT417 avalanche transistor are stacked. Each transistor has an collector-emitter breakdown voltage $BV_{CEO}$ of 100 V and a collector-base breakdown voltage $BV_{CBO}$ of 320 V. The circuit shown in Fig. 5 was simulated using LTSpice models. The Spice model of the FMMT417 was directly taken from the manufacture's website. The source resistance $R_c$ is 1 MΩ, characteristic impedance of the transmission line $Z_0$ is 50 Ω, source voltage $V_{cc}$ is 1.5kV.

The circuit may include a diode (not shown) connected in parallel with each transistor to clamp the voltage to ensure that the voltage across each transistor does not exceed its collector-base breakdown voltage. Doing so can increase the lifespan of the transistors and ensure that triggering occurs by the trigger signal.

The trigger signal may be provided by any suitable source. Preferably the trigger signal is generated by a TTL source or a microcontroller. In this example, the trigger signal comprises a pulse having a duration of 600 ns and a 5 V amplitude and pulse period (period of repetition) of 20 ms. It is advantageous to have a 5 V trigger signal because it is less than the emitter-base breakdown voltage of the transistors.

The pulse width of trigger signal is arranged to be longer than the pulse desired to be generated from the transmission line. The duration of 600 ns was chosen in this case to provide a safe margin to allow the whole transmission line to discharge.

The trigger signal repetition rate (pulse period) is limited by the time it takes for the open-circuit charged transmission line to charge up again to full capacity.

A transformer is disposed between the trigger signal generator and the base and emitter of the first transistor in the stack (i.e. the transistor furthest from the transmission line). This configuration means that the trigger pulse is floating, and therefore should be the same between the base and emitter of the first transistor no matter the voltage through the transistor and onto the load. As a result, the amplitude of the pulse at the load ought to increase linearly with the number of transistors in the stack. The transformer may be a 1-EMR-046 Gate Drive Transformer having a 1:1 winding ratio and high voltage isolation.

In use, the five stacked avalanche transistors are initially in their off-state, with each transistor having 300 V across them (i.e. $V_{cc}/n$). When a positive trigger signal is applied to the base of the first transistor Q1, Q1 is turned 'on' and places its collector voltage near ground potential. This results in the second transistor Q2 having twice the collector-emitter voltage, thus creating the desired condition in terms of overvolting and therefore causes a non-destructive avalanching of Q2 and places its collector near ground potential. This creates a sequential 'knock-on' effect on the next transistor in the chain resulting in the overvolting of the first avalanche transistors, Q1, to the final avalanche transistors, Q5 near the charged open circuit transmission line. When Q5 is turned 'on', a fast rise time is produced at the load (< 2 ns), therefore allowing the charged open circuit transmission line to discharge through the load producing a pulse with a width of $2T$ and a maximum amplitude of $V_{cc}/2$, if $R_L = Z_0$.

The pulse generating circuit 200 may thus be used to generate monopolar ultrashort electric field pulses.

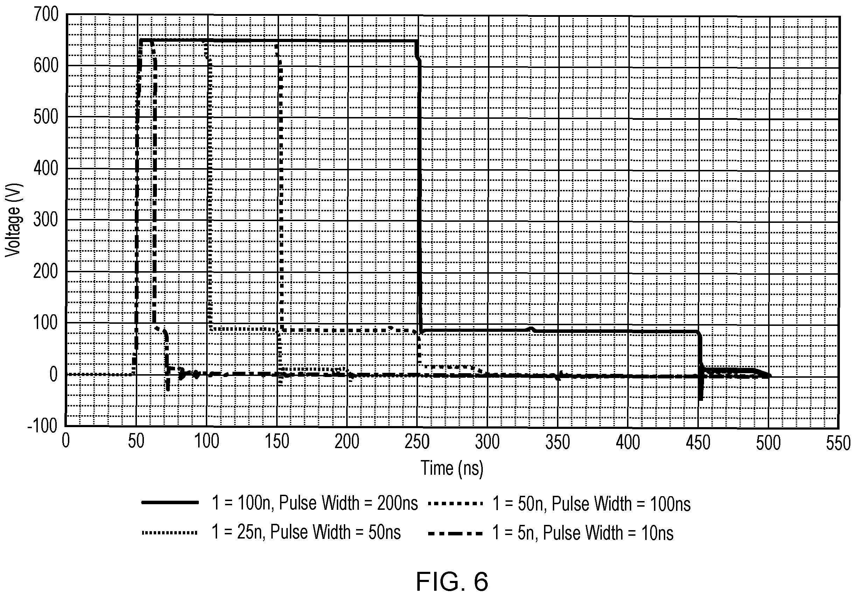

Fig. 6 is a graph showing voltage pulses obtained for a range of transmission line lengths. In Fig. 6, the transmission line lengths are characterised by the line delay $T$. The graph demonstrate that the transmission line length determines the pulse width of $2T$, i.e. transmission lines having line delays of 5 ns, 25 ns, 50 ns and 100 ns produce pulse widths of 10 ns, 50 ns, 100 ns and 200 ns respectively. Additionally, the rise times of all four pulses are the same and less than 2ns, which emphasises that the switching element, i.e. the five avalanche transistors, determines this factor.

The graph in Fig. 6 suggests that a 50 Q load does not match the transmission line characteristic impedance because secondary pulse of lower amplitude to the primary pulse is seen on each signal. This suggested an unmatched load due to reflection, i.e. $\Gamma \neq 0$. The inventors have realised that it is necessary to compensate for the impedance of the transistors in order to optimise the pulse generation circuit. In the example shown in Fig. 5, each individual transistor has an impedance of ~3 Q. Therefore, a total of ~15 Q is across the transistor stack. The reflection coefficient can thus be expressed as

$$\Gamma = \left(\frac{R_\Sigma - Z_0}{R_\Sigma + Z_0}\right) = \left(\frac{(R_L + nR_A) - Z_0}{(R_L + nR_A) + Z_0}\right)$$

wherein the $R_\Sigma$ is the total impedance of the circuit, and $R_A$ is the impedance of a signal avalanche transistor.

This explains the reflection observed in the pulses shown in Fig. 6, as $\Gamma$ = 0.13, and the amplitude of the reflection pulse is ~13% of the primary pulse ($R_L$ = 50 Ω, $nR_A$ = (3 Ω × 5) = 15 Ω and $Z_0$ = 50 Ω). The additional impedance of $nR_A$ also affects the DC component of the design, which can be rewritten as :

$$V_L = \left(\frac{R_L}{Z_0 + R_A + R_L}\right) V_{cc}$$

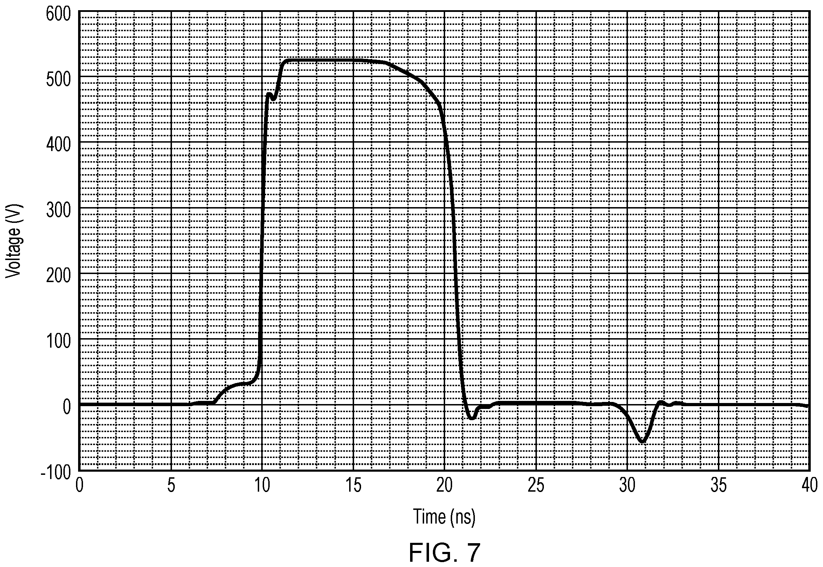

Taking this into account, the impedance of the load $R_L$ was adjusted to 35 Ω. This resulted in a single monopolar pulse at the load with zero reflection and no secondary pulse, as shown in Fig. 7.

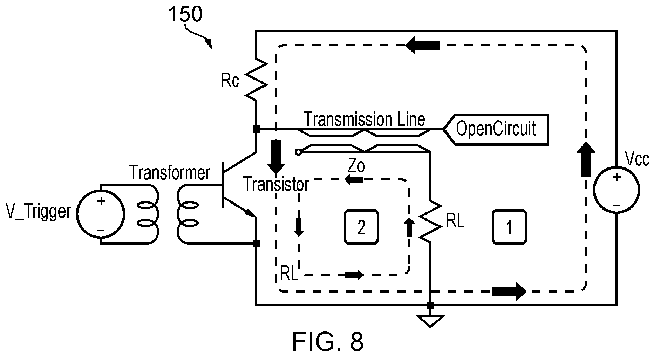

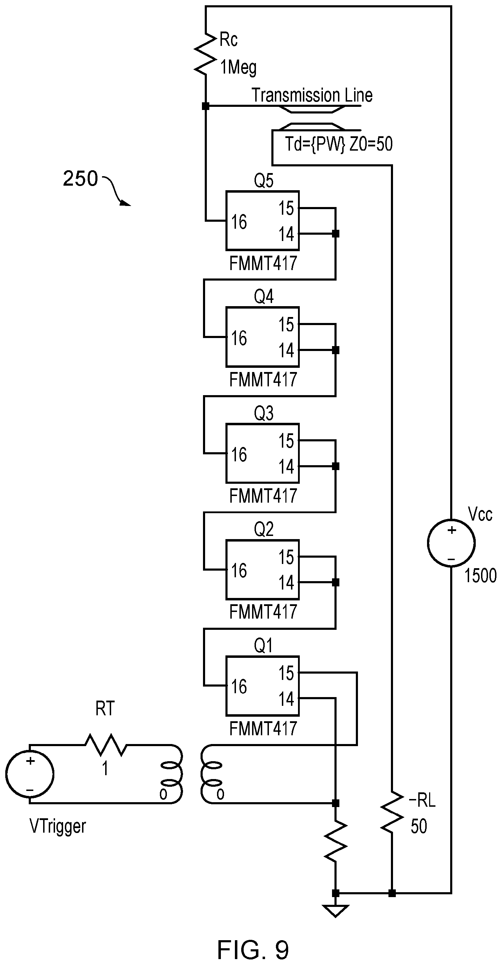

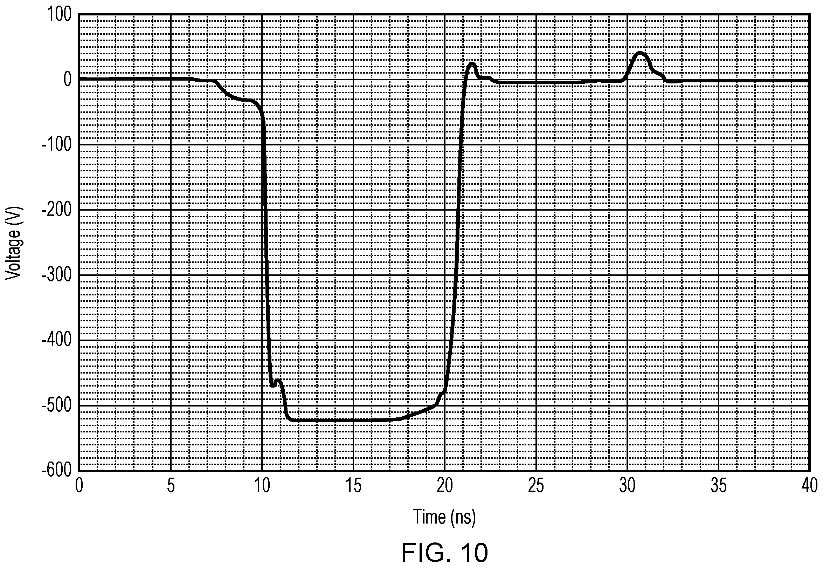

The circuit shown above in Figs. 4 and 5 is configured to generate positive monopolar pulses. However, the circuit can also be adapted to generate a negative monopolar pulse by changing the position in which the load is connected. Fig. 8 is a schematic diagram of a pulse generating circuit 150 that utilises an open circuit transmission line technique in combination with an avalanche transistor as a fast switching element similar to the circuit 100 in Fig. 4. The circuit 150 of Fig. 8 differs from the circuit of Fig. 4 in that the load $R_L$ is connected so that current flows in the opposite direction from Fig. 4 when the coaxial transmission line discharges. Fig. 9 shows a pulse generation circuit 250 that is an embodiment of the invention. The pulse generation circuit 250 is similar to the circuit shown in Fig. 5, except that the load $R_L$ is connected so that current flows in the opposite direction from Fig. 5 when the coaxial transmission line discharges. Fig. 10 shows a graph of a negative monopolar pulse observed with a matched 35 Ω load, obtained using the circuit 250 shown in Fig. 9.

In a development of the concepts discussed above, the pulse generating circuit can be configured as a bipolar pulse generating circuit. The operation of such a circuit can be identical to the monopolar designs in Figs. 4 and 9.

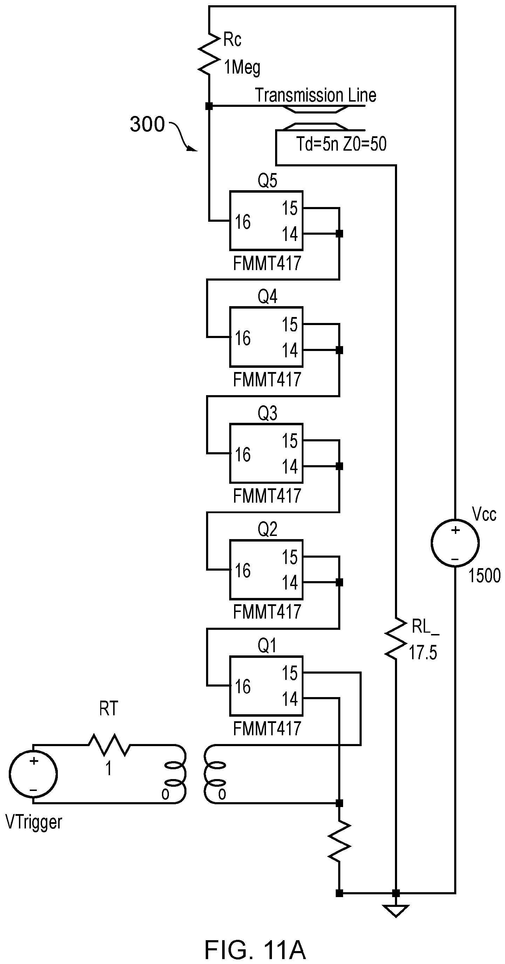

Fig. 11A is a schematic diagram of a pulse generation circuit 300 that is an embodiment of a bipolar pulse generating circuit 300. It is similar to the circuits shown in Figs. 4 and 9, except that the pulse is generated on two separate loads, which are marked at $R_{L+}$ and $R_{L-}$ in Fig. 11A. These load location correspond to the locations for the positive and negative pulses discussed above.

The bipolar pulse generating circuit 300 produces a bipolar pulse, as the voltage difference across $R_{L+}$ produces a positive pulse, where the voltage difference across $R_{L-}$ produces a negative pulse. When the circuit 300 is used, these pulses observed at on $R_{L+}$ and $R_{L-}$ simultaneously and are symmetrical, i.e. with the same pulse width, rise time, amplitude a repletion rate, but of different polarity.

As there are two loads in this circuit, the optimisation equations to reduce reflection must be revised. For a bipolar design the total load impedance, $R_{\Sigma L} = R_{L+} + R_{L-}$, is the impedance between the transmission line's outer conductor and the emitter of avalanche transistor Q1 and is the sum impedance of $R_{L+}$ and $R_{L-}$. The reflection coefficient can therefore be expressed using transmission line theory as:

$$\Gamma = \left(\frac{(R_{\Sigma L} + nR_A) - Z_0}{(R_{\Sigma L} + nR_A) + Z_0}\right) = \left(\frac{(R_{L-} + R_{L+} + nR_A) - Z_0}{(R_{L-} + R_{L+} + nR_A) + Z_0}\right)$$

$$\therefore if\ R_{\Sigma L} = R_{L-} + R_{L+} = Z_0 - nR_A\,, \qquad \Gamma = 0$$

Similarly, the peak-to-peak voltage $V_{\Sigma L}$ over the loads can be expressed using DC theory as:

$$V_{\Sigma L} = V_{L+} + V_{L-} = \left(\frac{R_{\Sigma L}}{Z_0 + R_A + R_{\Sigma L}}\right) V_{cc}$$

$$V_{L-} = \left(\frac{R_{L-}}{Z_0 + R_A + R_{\Sigma L}}\right) V_{cc}$$

$$V_{L+} = \left( \frac{R_{L+}}{Z_0 + R_A + R_{\Sigma L}} \right) V_{cc}$$

wherein $V_{L+}$ and $V_{L-}$ are the amplitudes of the positive and negative pulses respectively.

From the above, $R_{L+}$ and $R_{L-}$ values of 17.5 Q would produce a bipolar pulse of a single pulse with zero reflection ($\Gamma = 0$), and a simultaneous symmetrical pulse width of $2T$ and rise times < 2ns. Put another way, the bipolar pulse generating circuit 300 operates to create a single positive pulse of amplitude $V_{\Sigma L}$ between the transmission line's outer conductor and the emitter of avalanche transistor Q1, across $R_{\Sigma L}$ with a pulse width of 2T and zero reflection.

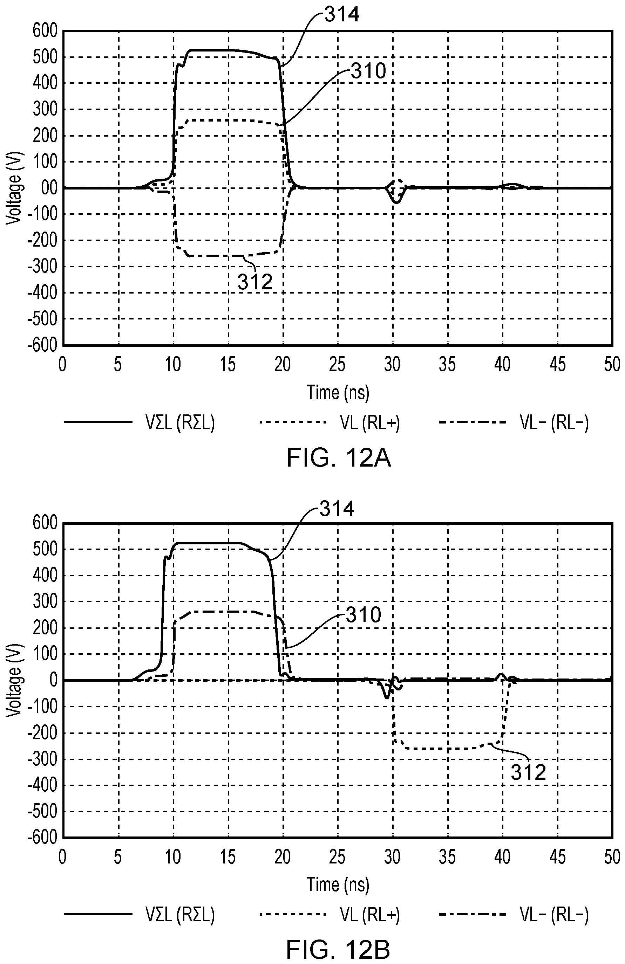

Fig. 12A is a graph that shows a pulse 310 observed at $R_{L+}$, a pulse 312 observed at $R_{L-}$, and a pulse 314 observed at $R_{\Sigma L}$. These observations verify the theory presented above. In Fig. 12A, a 5ns transmission line produces a 10ns pulse at all three loads with identical rise times (<2ns). As $R_{L+} = R_{L-} = 17.5\ \Omega$, there is no reflection, i.e.

$$\Gamma = \left( \frac{(R_{L-} + R_{L+} + nR_A) - Z_0}{(R_{L-} + R_{L+} + nR_A) + Z_0} \right) = \left( \frac{(17.5 + 17.5 + 15) - 50}{(17.5 + 17.5 + 15) + 50} \right) = 0$$

The magnitude of $V_{L+}$ and $V_{L-}$ is 262.5 V, so the peak-to-peak voltage $V_{\Sigma L}$ is 520 V, which is the same as the equivalent monopolar design.

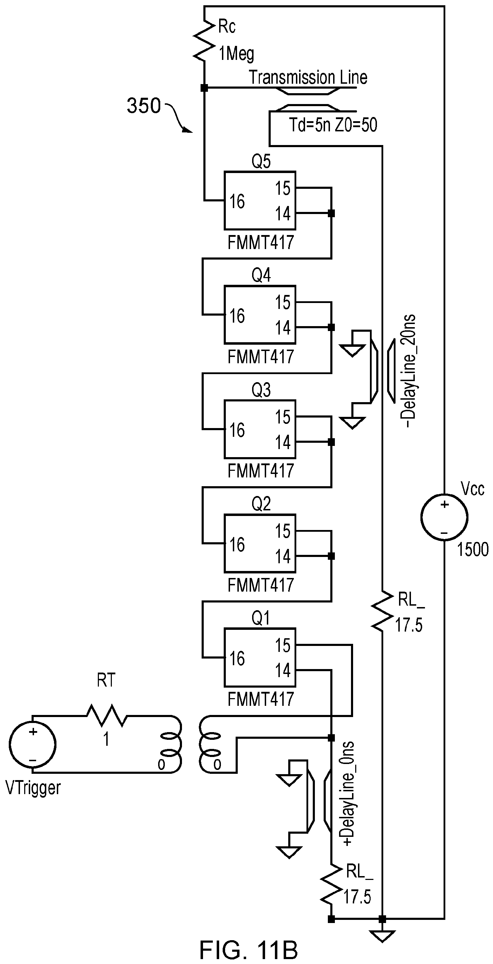

Fig. 11B is a schematic diagram of a bipolar pulse generation circuit 350 that is another embodiment of the invention. The circuit in Fig. 11B differs from Fig. 11A by providing a delay line before each of the loads ($R_{L+}$ and $R_{L-}$). Placing a delay line before one or both loads allows manipulation of a delay between the two pulses. A delayed pulse will follow a non-delayed paired pulse by the delay time minus pulse width. In Fig. 11B, a 20 ns delay line is placed before $R_{L-}$.

Fig. 12B is a graph similar to Fig. 12A that shows a pulse 310 observed at $R_{L+}$, a pulse 312 observed at $R_{L-}$, and a pulse 314 observed at $R_{\Sigma L}$. Fig. 12B confirms the effect of the introducing the delay line, as all the three pulses in Fig. 12B and there parameters are identical to the Fig. 12A. The only difference is that the negative pulse across $R_{L-}$ follows the positive pulse by 10ns (i.e. 20ns - 10 ns).

The bipolar pulse generation circuit configuration discussed herein is thus capable of producing:

- a symmetrical bipolar pulse, with positive and negative parts generated simultaneously or sequentially (i.e. with differing delays)
- zero reflection but adjustable $V_{L+}$ and $V_{L-}$ amplitudes, because the amplitudes are controlled by the ratio of $R_{L+}$ and $R_{L-}$ but the reflection will remain zero if $R_{\Sigma L} = R_{L-} + R_{L+} = Z_0 - nR_A$ condition is met.

In a further development, one or both of the delay lines may have an adjustable length that allows the introduced delay to be controlled. This may permit the separation of the positive and negative pulses to be adjusted on the fly, e.g. so that the instrument is capable of generating a variety of electroporation waveforms.

REFERENCES

[1] W. Meiling and F. Stary, Nanosecond pulse techniques. New York: Gordon and Breach, 1970, p. 304.
[2] Q. Yang, X. Zhou, Q.-g. Wang and M. Zhao, "Comparative analysis on the fast rising edge pulse source with two kinds of avalanche transistor," in Cross Strait Quad-Regional Radio Science and Wireless Technology Conference, Chengdu, 2013.
[3] G. Yong-sheng et al., "High-speed, high-voltage pulse generation using avalanche transistor," Review of Scientific Instruments, vol. 87, no. 5, p. 054708, 2016.

Claims

1. A bipolar pulse generating circuit for an electrosurgical generator, the bipolar pulse generating circuit comprising:

a voltage source connectable to a load via a switching element;
a coaxial transmission line having an inner conductor separated from an outer conductor by a dielectric material, wherein the inner conductor has a first end connected between an input of the switching element and the voltage

**EP 4 069 117 B1**

source and a second end in an open circuit condition, whereby the coaxial transmission line is charged by the voltage source when the switching element is in an OFF state and to be discharged when the switching element is in an ON state;

a first output connectable to the load, wherein the first output is located between an output of the switching element and ground to support a positive pulse when the coaxial transmission line discharges; and

a second output connectable to the load, wherein the second output is located between the outer conductor of the coaxial transmission line and ground to support a negative pulse when the coaxial transmission line discharges,

wherein the impedance of the coaxial transmission line is configured to match a sum of (i) the impedance the switching element, (ii) the impedance of the load at the first output, and (iii) the impedance of the load at the second output,

wherein a delay line is connected to either the first output or the second output, whereby supply of the positive pulse and negative pulse at the first output and second output occurs sequentially.

2. A bipolar pulse generating circuit according to claim 1, wherein the delay line has an adjustable length.

3. A bipolar pulse generating circuit according to claim 1 or 2, wherein the delay line is another length of coaxial transmission line.

4. A bipolar pulse generating circuit according to any preceding claim, wherein the switching element comprises:

a plurality of series connected avalanche transistors; and
a trigger pulse generator configured to generate a trigger pulse to activate the plurality of series connected avalanche transistors.

5. A bipolar pulse generating circuit according to claim 4, wherein the trigger pulse generator comprises a TTL device.

6. A bipolar pulse generating circuit according to claim 4 or 5, wherein the trigger pulse has a voltage less than the emitter-base breakdown voltage of each of the plurality of avalanche transistors.

7. A bipolar pulse generating circuit according to any one of claims 4 to 6, wherein the trigger pulse generator is connected to the plurality of series connected avalanche transistors via a transformer.

8. A bipolar pulse generating circuit according to any one of claims 4 to 7, wherein the trigger pulse is applied between the collector and emitter of a first transistor of the plurality of series connected avalanche transistors.

9. A bipolar pulse generating circuit according to claim 8, wherein the first transistor is furthest from the coaxial transmission line.

10. A bipolar pulse generating circuit according to any one of claims 4 to 9, wherein a diode is connected in parallel with each of the plurality of series connected avalanche transistors to clamp the voltage across each transistor to less than its collector-base breakdown voltage.

11. A bipolar pulse generating circuit according to any one of claims 4 to 10, wherein each transistor in the plurality of series connected avalanche transistors is identical.

12. A bipolar pulse generating circuit according to any preceding claim, wherein the coaxial transmission line has a length selected to provide a line delay equal to or less than 5 ns.

13. A bipolar pulse generating circuit according to any preceding claim, wherein the coaxial transmission line is charged by the voltage source through a resistor.

14. A bipolar pulse generating circuit according to any preceding claim, wherein the load is an electrosurgical instrument.

15. An electrosurgical generator having a bipolar pulse generating circuit according to any preceding claim.

**Patentansprüche**

1. Schaltung zur Erzeugung bipolarer Impulse für einen elektrochirurgischen Generator, wobei die Schaltung zur Erzeugung bipolarer Impulse Folgendes umfasst:

   eine Spannungsquelle, die über ein Schaltelement mit einer Last verbindbar ist;
   eine koaxiale Übertragungsleitung mit einem inneren Leiter, der von einem äußeren Leiter durch ein dielektrisches Material getrennt ist, wobei der innere Leiter ein erstes Ende, das zwischen einen Eingang des Schaltelements und die Spannungsquelle geschaltet ist, und ein zweites Ende in einem Leerlaufzustand aufweist, wobei die koaxiale Übertragungsleitung durch die Spannungsquelle geladen wird, wenn das Schaltelement in einem AUS-Zustand ist, und entladen wird, wenn das Schaltelement in einem EIN-Zustand ist;
   einen ersten Ausgang, der mit der Last verbindbar ist, wobei der erste Ausgang zwischen einem Ausgang des Schaltelements und Masse angeordnet ist, um einen positiven Impuls zu unterstützen, wenn die Übertragungsleitung entladen wird; und
   einen zweiten Ausgang, der mit der Last verbindbar ist, wobei der zweite Ausgang zwischen dem äußeren Leiter der koaxialen Übertragungsleitung und Masse angeordnet ist, um einen negativen Impuls zu unterstützen, wenn die Übertragungsleitung entladen wird,
   wobei die Impedanz der koaxialen Übertragungsleitung ausgelegt ist, um der Summe aus (i) der Impedanz des Schaltelements, (ii) der Impedanz der Last am ersten Ausgang und (iii) der Impedanz der Last am zweiten Ausgang angepasst zu sein, wobei eine Verzögerungsleitung mit entweder dem ersten Ausgang oder dem zweiten Ausgang verbunden ist, wodurch die Bereitstellung des positiven Impulses und des negativen Impulses am ersten Ausgang und am zweiten Ausgang nacheinander erfolgt.

2. Schaltung zur Erzeugung bipolarer Impulse nach Anspruch 1, wobei die Verzögerungsleitung eine einstellbare Länge aufweist.

3. Schaltung zur Erzeugung bipolarer Impulse nach Anspruch 1 oder 2, wobei die Verzögerungsleitung eine weitere Länge der koaxialen Übertragungsleitung ist.

4. Schaltung zur Erzeugung bipolarer Impulse nach einem der vorangegangenen Ansprüche, wobei das Schaltelement Folgendes umfasst:

   eine Vielzahl von in Serie geschalteten Avalanchetransistoren; und
   einen Triggerimpulsgenerator, der ausgelegt ist, um einen Triggerimpuls zu erzeugen, um die Vielzahl von in Serie geschalteten Avalanchetransistoren zu aktivieren.

5. Schaltung zur Erzeugung bipolarer Impulse nach Anspruch 4, wobei der Triggerimpulsgenerator eine TTL-Vorrichtung umfasst.

6. Schaltung zur Erzeugung bipolarer Impulse nach Anspruch 4 oder 5, wobei der Triggerimpuls eine niedrigere Spannung aufweist als die Emitter-Basis-Durchbruchspannung jedes der Vielzahl von Avalanchetransistoren.

7. Schaltung zur Erzeugung bipolarer Impulse nach einem der Ansprüche 4 bis 6, wobei der Triggerimpulsgenerator über einen Wandler mit der Vielzahl von in Serie geschalteter Avalanchetransistoren verbunden ist.

8. Schaltung zur Erzeugung bipolarer Impulse nach einem der Ansprüche 4 bis 7, wobei der Triggerimpuls zwischen dem Kollektor und Emitter eines ersten Transistors der Vielzahl von in Serie geschalteten Avalanchetransistoren angelegt wird.

9. Schaltung zur Erzeugung bipolarer Impulse nach Anspruch 8, wobei der erste Transistor am weitesten von der koaxialen Übertragungsleitung entfernt ist.

10. Schaltung zur Erzeugung bipolarer Impulse nach einem der Ansprüche 4 bis 9, wobei eine Diode parallel zu jedem der Vielzahl von in Reihe verbundenen Avalanchetransistoren geschaltet ist, um die Spannung an jedem Transistor auf unter die Kollektor-Basis-Durchbruchspannung zu klemmen.

11. Schaltung zur Erzeugung bipolarer Impulse nach einem der Ansprüche 4 bis 10, wobei alle Transistoren in der Vielzahl von in Serie geschalteten Avalanchetransistoren identisch sind.

**12.** Schaltung zur Erzeugung bipolarer Impulse nach einem der vorangegangenen Ansprüche, wobei die koaxiale Übertragungsleitung eine Länge aufweist, die ausgewählt ist, um eine Leitungsverzögerung von gleich oder weniger als 5 ns bereitzustellen.

**13.** Schaltung zur Erzeugung bipolarer Impulse nach einem der vorangegangenen Ansprüche, wobei die koaxiale Übertragungsleitung über einen Widerstand von der Spannungsquelle geladen wird.

**14.** Schaltung zur Erzeugung bipolarer Impulse nach einem der vorangegangenen Ansprüche, wobei die Last ein elektrochirurgisches Instrument ist.

**15.** Elektrochirurgischer Generator mit einer Schaltung zur Erzeugung bipolarer Impulse nach einem der vorangegangenen Ansprüche.

**Revendications**

**1.** Circuit de génération d'impulsion bipolaire pour un générateur électrochirurgical, le circuit de génération d'impulsion bipolaire comprenant :

    une source de tension pouvant être connectée à une charge via un élément de commutation ;
    une ligne de transmission coaxiale présentant un conducteur interne séparé d'un conducteur externe par un matériau diélectrique, dans lequel le conducteur interne présente une première extrémité connectée entre une entrée de l'élément de commutation et la source de tension et une seconde extrémité dans un état de circuit ouvert, moyennant quoi la ligne de transmission coaxiale est chargée par la source de tension lorsque l'élément de commutation est dans un état ARRÊT et devant être déchargée lorsque l'élément de commutation est dans un état MARCHE ;
    une première sortie pouvant être connectée à la charge, dans lequel la première sortie est située entre une sortie de l'élément de commutation et la terre pour supporter une impulsion positive lorsque la ligne de transmission coaxiale se décharge ; et
    une seconde sortie pouvant être connectée à la charge, dans lequel la seconde sortie est située entre le conducteur externe de la ligne de transmission coaxiale et la terre pour supporter une impulsion négative lorsque la ligne de transmission coaxiale se décharge,
    dans lequel l'impédance de la ligne de transmission coaxiale est configurée pour correspondre à une somme (i) de l'impédance de l'élément de commutation, (ii) de l'impédance de la charge au niveau de la première sortie, et (iii) de l'impédance de la charge au niveau de la seconde sortie,
    dans lequel une ligne de retard est connectée à la première sortie ou à la seconde sortie, moyennant quoi la fourniture de l'impulsion positive et de l'impulsion négative au niveau de la première sortie et de la seconde sortie survient séquentiellement.

**2.** Circuit de génération d'impulsion bipolaire selon la revendication 1, dans lequel la ligne de retard présente une longueur ajustable.

**3.** Circuit de génération d'impulsion bipolaire selon la revendication 1 ou 2, dans lequel la ligne de retard est une autre longueur de ligne de transmission coaxiale.

**4.** Circuit de génération d'impulsion bipolaire selon l'une quelconque des revendications précédentes, dans lequel l'élément de commutation comprend :

    une pluralité de transistors à avalanche connectés en série ; et
    un générateur d'impulsion de déclenchement configuré pour générer une impulsion de déclenchement afin d'activer la pluralité de transistors à avalanche connectés en série.

**5.** Circuit de génération d'impulsion bipolaire selon l'une quelconque des revendications précédentes, dans lequel le générateur d'impulsion de déclenchement comprend un dispositif TTL.

**6.** Circuit de génération d'impulsion bipolaire selon la revendication 4 ou 5, dans lequel l'impulsion de déclenchement présente une tension inférieure à la tension de claquage de base-émetteur de chacun de la pluralité de transistors à avalanche.

7. Circuit de génération d'impulsion bipolaire selon l'une quelconque des revendications 4 à 6, dans lequel le générateur d'impulsion de déclenchement est connecté à la pluralité de transistors à avalanche connectés en série via un transformateur.

8. Circuit de génération d'impulsion bipolaire selon l'une quelconque des revendications 4 à 7, dans lequel l'impulsion de déclenchement est appliquée entre le collecteur et l'émetteur d'un premier transistor de la pluralité de transistors à avalanche connectés en série.

9. Circuit de génération d'impulsion bipolaire selon la revendication 8, dans lequel le premier transistor est le plus éloigné de la ligne de transmission coaxiale.

10. Circuit de génération d'impulsion bipolaire selon l'une quelconque des revendications 4 à 9, dans lequel une diode est connectée en parallèle avec chacun de la pluralité de transistors à avalanche connectés en série pour fixer la tension aux bornes de chaque transistor à moins que sa tension de claquage de base-collecteur.

11. Circuit de génération d'impulsion bipolaire selon l'une quelconque des revendications 4 à 10, dans lequel chaque transistor de la pluralité de transistors à avalanche connectés en série est identique.

12. Circuit de génération d'impulsion selon l'une quelconque des revendications précédentes, dans lequel la ligne de transmission coaxiale présente une longueur choisie pour fournir un retard de ligne égal ou inférieur à 5 ns.

13. Circuit de génération d'impulsion bipolaire selon l'une quelconque des revendications précédentes, dans lequel la ligne de transmission coaxiale est chargée par la source de tension à travers une résistance.

14. Circuit de génération d'impulsion bipolaire selon l'une quelconque des revendications précédentes, dans lequel la charge est un instrument électrochirurgical.

15. Générateur électrochirurgical présentant un circuit de génération d'impulsion bipolaire selon l'une quelconque des revendications précédentes.

FIG. 1

FIG. 2

FIG. 3A

FIG. 3B

FIG. 4

FIG. 5

FIG. 6

FIG. 7

FIG. 8

FIG. 9

FIG. 10

EP 4 069 117 B1

FIG. 11A

FIG. 11B

FIG. 12A

FIG. 12B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019185331 A1

**Non-patent literature cited in the description**

- **TAMPIERI DANIELE.** Avalanche transistor. *Wikipedia,* 30 November 2019
- **WERNER B HERDEN.** Application of avalanche transistors to circuits with a long mean time to failure. *IEEE Transactions on instrumentation and measurement,* 01 June 1976, vol. IM-25 (2), 152-160
- **W. MEILING ; F. STARY.** Nanosecond pulse techniques. Gordon and Breach, 1970, 304
- **Q. YANG ; X. ZHOU ; Q.-G. WANG ; M. ZHAO.** Comparative analysis on the fast rising edge pulse source with two kinds of avalanche transistor. *Cross Strait Quad-Regional Radio Science and Wireless Technology Conference,* 2013
- **G. YONG-SHENG et al.** High-speed, high-voltage pulse generation using avalanche transistor. *Review of Scientific Instruments,* 2016, vol. 87 (5), 054708